(19) Europäisches Patentamt
European Patent Office
Office européen des brevets

(11) **EP 4 542 562 A2**

(12) **EUROPEAN PATENT APPLICATION**

(43) Date of publication:
**23.04.2025 Bulletin 2025/17**

(21) Application number: **24202208.5**

(22) Date of filing: **24.09.2024**

(51) International Patent Classification (IPC):
**G16H 20/30** (2018.01)    **G16H 30/40** (2018.01)
**G16H 50/70** (2018.01)

(52) Cooperative Patent Classification (CPC):
**G16H 20/30; G16H 30/40; G16H 50/70**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **27.09.2023 US 202363585743 P**
          **15.02.2024 US 202418442667**

(71) Applicant: **Siemens Healthineers AG**
**91301 Forchheim (DE)**

(72) Inventors:
• **ABDISHEKTAEI, Mohammad**
  **Charlottesville, VA 22903 (US)**
• **YEREBAKAN, Halid**
  **Carmel, IN 46033 (US)**
• **HERMOSILLO VALADEZ, Gerardo**
  **West Chester, PA 19382 (US)**

(74) Representative: **Horn Kleimann Waitzhofer**
**Schmid-Dreyer**
**Patent- und Rechtsanwälte PartG mbB**
**Theresienhöhe 12**
**80339 München (DE)**

(54)    **ANATOMICAL POSITIONING FRAMEWORK**

(57)    A framework for anatomical positioning. In accordance with one aspect, input text is mapped into normalized coordinates using an artificial neural network. A location in a target image that corresponds to the normalized coordinates is determined and presented. In accordance with another aspect, a user selection of a point-of-interest in a medical image is received. A context set of points nearest to the point-of-interest is determined. A prompt containing the point-of-interest and context set of points is constructed. A large language model may then generate text data associated with the point-of-interest in response to the prompt

Fig. 1

EP 4 542 562 A2

## Description

CROSS-REFERENCE TO RELATED APPLICATION

The present application claims the benefit of U.S. provisional application no. 63/585,743 filed September 27, 2023 the entire contents of which are herein incorporated by reference.

TECHNICAL FIELD

The present disclosure generally relates to data processing, and more particularly to a framework for anatomical positioning.

BACKGROUND

A written radiology report is the primary work product of a diagnostic radiologist and acts as the main method of communicating results of a radiologic study or procedure, including findings in medical images. Radiology reports are intended to be reused after image reading is complete. When the reports are re-used, clinicians often have to manually find the corresponding entries in the images. These entries may include the findings in organs and in sub-tissue levels, and are usually described with respect to their locations relative to some known anatomical landmarks.

This process may be automated by automatically mapping the text entries in the radiology reports to the anatomical landmarks in the images. Navigating to the specific anatomical landmarks is not a trivial problem because (1) the anatomical landmarks may appear in different forms and orders; and (2) storing the information in unstructured text limits the ability to process data on a large scale. For example, identifying patients with liver disease may require taking all type of mentions (e.g., "liver", "hepatic") into consideration.

Previous methods seek to identify longitudinal range of an image given Digital Imaging and Communications in Medicine (DICOM) text input. There are, however, some limitations to these methods. Estimation of the range in only one-dimensional and there is limited applicability since only the header data is used. It may not work well on natural language sentences present in the radiology reports.

Parsing methods may be considered as baselines for associating text input to anatomical coordinates, since the image identifiers (e.g., image and series numbers) mentioned in the text follows a well-structured pattern. However, each keyword must be manually engineered and extracted, thereby restricting its application. Another approach is to use the locations of the anatomical landmarks in the images identified by segmentation or landmarking methods. However, the scope is limited to only landmarks and organs with defined structure.

SUMMARY

Described herein is a framework for anatomical positioning. In accordance with one aspect, input text is mapped into normalized coordinates using an artificial neural network. A location in a target image that corresponds to the normalized coordinates is determined and presented. In accordance with another aspect, a user selection of a point-of-interest in a medical image is received. A context set of points nearest to the point-of-interest is determined. A prompt containing the point-of-interest and context set of points is constructed. A large language model may then generate text data associated with the point-of-interest in response to the prompt.

BRIEF DESCRIPTION OF THE DRAWINGS

A more complete appreciation of the present disclosure and many of the attendant aspects thereof will be readily obtained as the same becomes better understood by reference to the following detailed description when considered in connection with the accompanying drawings.

FIG. 1 shows a block diagram illustrating an exemplary system;

FIG. 2 is a flow chart of an exemplary method of estimating an image location from text input;

FIG. 3 is schematic diagram of the exemplary method of estimating an image location from text input;

FIG. 4 is a flow chart of an exemplary method of determining text data associated with a point-of-interest in a medical image; and

FIG. 5 is schematic diagram of the exemplary method of determining text data associated with a point-of-interest in a medical image.

DETAILED DESCRIPTION

In the following description, numerous specific details are set forth such as examples of specific components, devices, methods, etc., in order to provide a thorough understanding of implementations of the present framework. It will be apparent, however, to one skilled in the art that these specific details need not be employed to practice implementations of the present framework. In other instances, well-known materials or methods have not been described in detail in order to avoid unnecessarily obscuring implementations of the present framework. While the present framework is susceptible to various modifications and alternative forms, specific embodiments thereof are shown by way of example in the drawings and will herein be described in detail. It should be understood, however, that there is no intent to limit the invention to the particular forms disclosed, but on the contrary, the intention is to cover all modifications, equivalents, and alternatives falling within the spirit and scope of the invention. Furthermore, for ease of understanding, certain method steps are delineated as separate steps; however, these separately delineated steps should not be construed as necessarily order dependent in their performance. Independent of the grammatical term usage, individuals with male, female or other gender identities are included within the term.

Unless stated otherwise as apparent from the following discussion, it will be appreciated that terms such as "segmenting," "generating," "registering," "determining," "aligning," "positioning," "processing," "computing," "selecting," "estimating," "detecting," "tracking" or the like may refer to the actions and processes of a computer system, or similar electronic computing device, that manipulates and transforms data represented as physical (e.g., electronic) quantities within the computer system's registers and memories into other data similarly represented as physical quantities within the computer system memories or registers or other such information storage, transmission or display devices. Embodiments of the methods described herein may be implemented using computer software. If written in a programming language conforming to a recognized standard, sequences of instructions designed to implement the methods can be compiled for execution on a variety of hardware platforms and for interface to a variety of operating systems. In addition, implementations of the present framework are not described with reference to any particular programming language. It will be appreciated that a variety of programming languages may be used.

For brevity, an image, or a portion thereof (e.g., a region of interest (ROI) in the image) corresponding to an object (e.g., a tissue, an organ, a tumor, etc., of a subject (e.g., a patient, etc.)) may be referred to as an image, or a portion of thereof (e.g., an ROI) of or including the object, or the object itself. For instance, an ROI corresponding to the image of a lung or a heart may be described as that the ROI includes a lung or a heart. As another example, an image of or including a chest may be referred to a chest image, or simply a chest. For brevity, that a portion of an image corresponding to an object is processed (e.g., extracted, segmented) may be described as the object is processed. For instance, that a portion of an image corresponding to a lung is extracted from the rest of the image may be described as that the lung is extracted.

There is a growing interest in establishing correspondence between radiology reports and corresponding images. Typically, a region-of-interest is found through attention maps or neural network activations corresponding to textual information. Instead of using attention maps and network activations directly, the present framework uses semantic coordinates for anatomical relationships. The present framework maps information between anatomical and textual domains. The anatomical information may include the position of anatomical landmarks in a normalized coordinate system. The textual information may include text entries in the radiology reports corresponding to the anatomical landmarks. The present framework may be used in either or both forward and backward directions.

In the forward usage of the method, the framework may estimate the position of a specific anatomical landmark in terms of normalized coordinates using text input entries. Instead of specific organ labels in the output, a continuous normalized coordinate system that maps to a standard human body is used. This kind of mapping may describe the anatomy in various granularities. In the backward usage of the method, the semantic meaning of the presented normalized coordinates may be identified using a large-language-model (LLM) with knowledge of human anatomy. Anatomical landmarks may be identified using only positional information from the image data.

Keyword level detectors of anatomies may work only a certain degree. Machine learning models, on the other hand, may provide better generalization. The present framework utilizes the existing landmarks and LLMs to generate more precise estimations. These and other exemplary advantages and features will be described in more details in the following description.

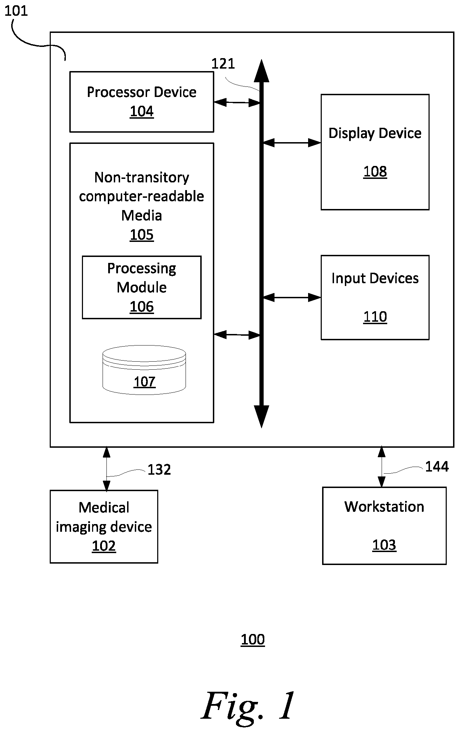

FIG. 1 is a block diagram illustrating an exemplary system 100. The system 100 includes a computer system 101 for implementing the framework as described herein. In some implementations, computer system 101 operates as a standalone device. In other implementations, computer system 101 may be connected (e.g., using a network) to other machines, such as medical imaging device 102 and workstation 103. In a networked deployment, computer system 101 may operate in the capacity of a server (e.g., in a server-client user network environment, a client user machine in server-client user network environment, or as a peer machine in a peer-to-peer (or distributed) network environment).

In one implementation, computer system 101 includes a processor device or central processing unit (CPU) 104 coupled to one or more non-transitory computer-readable media 105 (e.g., computer storage or memory device), display device 108 (e.g., monitor) and various input devices 110 (e.g., mouse, touchpad or keyboard) via an input-output interface 121. Computer system 101 may further include support circuits such as a cache, a power supply, clock circuits and a communications bus. Various other peripheral devices, such as additional data storage devices and printing devices, may also be connected to the computer system 101.

The present technology may be implemented in various forms of hardware, software, firmware, special purpose processors, or a combination thereof, either as part of the microinstruction code or as part of an application program or software product, or a combination thereof, which is executed via the operating system. In some implementations, the techniques described herein are implemented as computer-readable program code tangibly embodied in one or more non-transitory computer-readable media 105. In particular, the present techniques may be implemented by a processing module 106 and database 107.

Non-transitory computer-readable media 105 may include random access memory (RAM), read-only memory (ROM), magnetic floppy disk, flash memory, and other types of memories, or a combination thereof. The computer-readable program code is executed by processor device 104 to process data provided by, for example, medical imaging device 102. As such, the computer system 101 is a general-purpose computer system that becomes a specific-purpose computer system when executing the computer-readable program code. The computer-readable program code is not intended to be limited to any particular programming language and implementation thereof. It will be appreciated that a variety of programming languages and coding thereof may be used to implement the teachings of the disclosure contained herein. The same or different computer-readable media 105 may be used for storing a database, including, but not limited to, image datasets, a knowledge base, individual subject data, electronic medical records (EMRs), diagnostic reports (or documents) for subjects, or a combination thereof.

Medical imaging device 102 acquires medical image data 132. Such medical image data 132 may be processed by processing module 106. Medical imaging device 102 may be a radiology scanner and/or appropriate peripherals (e.g., keyboard and display device) for acquiring, collecting and/or storing such medical image data 132. Medical imaging device 102 may acquire medical image data 132 from a subject or patient by using techniques such as high-resolution computed tomography (HRCT), magnetic resonance (MR) imaging, computed tomography (CT), helical CT, X-ray, angiography, positron emission tomography (PET), fluoroscopy, ultrasound, single photon emission computed tomography (SPECT), or a combination thereof. Other types of imaging modalities are also useful. Medical imaging device 102 may be controlled using a medical imaging software.

Medical image data 132 represents a medical image (or in certain examples more than one medical image). For example, medical image data 132 may include an array or list of pixel or voxel values. When processed by suitable image viewing software, the medical image data 132 results in a rendering of the medical image (or medical images) that it represents. The image file containing the medical image data 132 may further include one or more attributes and attribute values. A specific example of an image file is a Digital Imaging and Communications in Medicine (DICOM) file. The one or more attribute values are separate to and distinct from the medical image data 132, and instead comprise a text string indicating content of the medical image data 132.

Such attribute values may, in some examples, be referred to as metadata of the image file. In some examples, the part of the image file that stores the attributes and attribute values may be referred to as a header of the image file, and the attributes and attribute values may be referred to as header data of the image file.

Workstation 103 may include a computer and appropriate peripherals, such as a keyboard and display device, and can be operated in conjunction with the entire system 100. For example, workstation 103 may communicate with medical imaging device 102 so that the medical image data 132 can be presented or displayed at the workstation 103. The workstation 103 may communicate directly with the computer system 101 to display processed data and/or output results 144. The workstation 103 may include a graphical user interface to receive user input via an input device (e.g., keyboard, mouse, touch screen, voice or video recognition interface, etc.) to manipulate visualization and/or processing of the data.

It is to be further understood that, because some of the constituent system components and method steps depicted in the accompanying figures can be implemented in software, the actual connections between the systems components (or the process steps) may differ depending upon the manner in which the present framework is programmed. Given the teachings provided herein, one of ordinary skill in the related art will be able to contemplate these and similar implementations or configurations of the present framework.

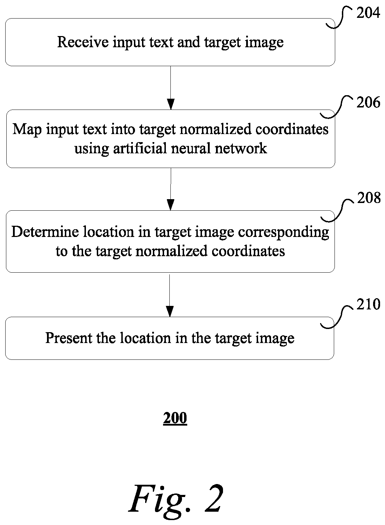

FIG. 2 is a flow chart of an exemplary method 200 of estimating at image location from text input. It should be understood that the steps of the method 200 may be performed in the order shown or a different order. Additional, different, or fewer steps may also be provided. Further, the method 200 may be implemented with the system 100 of FIG. 1, a different system, or a combination thereof.

At 204, processing module 106 receives input text and a target image of a patient. The input text may be extracted from a radiology report that is associated with the target image. The input may also be provided by a user via a user interface. The target image is a medical image of a current patient acquired by, for example, medical imaging device 102. The target image may, for example, a PET, MR, SPECT, X-ray or other type of medical image.

In some implementations, processing module 106 splits the radiology report into different parts, wherein each part corresponds to a specific location in the target image. The report may first be pre-processed to, for example, remove stop words (e.g., "a", "and", "to") and punctuation that add noise to data. Tokenization techniques (e.g., tokenization) may then be applied to split the radiology report into meaningful elements, such as sentences or tokens. Tokenization techniques may include, for example, rule-based methods or artificial neural network-based methods. Each token may then be processed as input text by the trained artificial neural network. In some implementations, the tokens are optionally filtered to provide only valid tokens as input text into the trained artificial neural network. The tokens may be filtered by using a trained classifier to determine whether each token describes an anatomical feature or not. Such classifier is trained separately from the artificial neural network. The user interface may provide an option for the user to select the filter for the input text.

In some implementations, the input text is selected by a user via a user interface (e.g., graphical user interface) displayed at, for example, workstation 103. The user may select one or more words (e.g., phrase, sentence) in, for example, a radiology report, as input text. A free text box may also be provided to allow the user to enter the input text. The user may create a hyperlink at the selected input text. The hyperlink may also be automatically inserted at the input text by the processing module 106.

At 206, processing module 106 maps the input text into target normalized coordinates using an artificial neural network. As discussed previously, the input text may be extracted from a radiology report manually or automatically by the processing module 106. Additionally, the target normalized coordinates may be defined with reference to a selected anatomical atlas of a standard body or part thereof.

The artificial neural network may be trained with a training set of keywords and corresponding normalized coordinates. The artificial neural network may be trained to map any input text to normalized coordinates. The artificial neural network may include, for example, deep convolutional neural networks, recursive neural networks, recurrent neural network, multilayer perceptron, long short-term memory, shallow neural network, or sequence-to-sequence model. Other types of artificial neural networks are also useful.

Keywords in the training set are one or more words that describe anatomical features (e.g., landmarks or organs) in an anatomical atlas (e.g., human body atlas). Examples of such keywords include, but are not limited to, upper lobe, pulmonary artery, pulmonary vein, middle lobe, aorta, aortic arch, left ventricle, right atrium, kidney center, and so forth. The keywords may describe the respective anatomical feature at different levels of granularities (e.g., body, chest, heart, tricuspid valve). Each keyword maps to corresponding normalized coordinates of a landmark or bounding box of an organ in an anatomical atlas.

The anatomical atlas is a reference image that represents a standard body (e.g., human body) or part thereof. The anatomical atlas may be stored in the database 107 and accessed by the processing module 106. For example, the database 107 may include one or more anatomical atlases that represent various types of people with respective characteristics (e.g., body type, height, weight, gender, age, race). The processing module 106 may select the anatomical atlas that most closely corresponds to the particular set of characteristics of the patient currently being imaged.

The normalized coordinate system is a continuous coordinate system that is scaled to a pre-defined bounding box (e.g., 0 to 1 scale for each dimension) or atlas patient image frame with a pre-defined origin. Such normalization may advantageously improve the neural network training in general. The normalized coordinates may be two-dimensional or three-dimensional. In some implementations, each anatomical location coordinate is scaled between 0 and 1 by dividing the voxel location by the volume size (i.e., width, height, breadth) of the anatomical atlas. Therefore, location (0,0,0) may be the bottom right anterior corner of the bounding box predefined for the normalized coordinate system, while location (1,1,1) may be the top left corner of the bounding box.

In other implementations, normalization is performed by subtraction of pre-defined landmark coordinates. In this normalization method, a known landmark is pre-defined as the origin of the coordinate system. Such pre-defined landmark becomes a carina with coordinates (0, 0, 0). Any other coordinates in the initial frame of the atlas (or target image) may be computed by subtracting the offset of the pre-defined landmark. In such case, distances are specified in standard units of measurements (e.g., millimeters), which may be easier to use in the real world.

At 208, processing module 106 determines the location in the target image that corresponds to the target normalized coordinates estimated by the trained artificial neural network. In some implementations, the corresponding location is determined by performing a search to find the location within the target image with normalized coordinates that match the target normalized coordinates. To perform the search, the framework may start from a first image location within the target image, normalize the coordinates of that first image location, and estimate where to find the target normalized coordinates. A trained regression neural network may be used to normalize the coordinates. The regression neural network may include, for example, an 8-layer residual network. Other types of artificial neural networks are also useful. The input of the neural network may be the first image location and associated image intensity data, and the output of the neural network is the normalized coordinates of the first image location.

In other implementations, landmark detectors are used to determine the location in the target image corresponding to the target normalized coordinates. Landmarks may be assigned to the normalized coordinate space and the corresponding location may be computed from nearby landmarks using, for example, linear interpolation methods.

At 210, processing module 106 presents the location in the target image. The determined location may be highlighted in the target image and displayed at, for example, workstation 103. In some implementations, when the user selects the hyperlink at the input text, the user is directed to that corresponding location in the target image. The user may select the hyperlink using, for example, a mouse, cursor or other user interface component. The corresponding location in the target image may be highlighted and displayed, for instance, in a window next to the radiology report whenever the hyperlink at the input text is selected. Other ways of presenting the location are also possible.

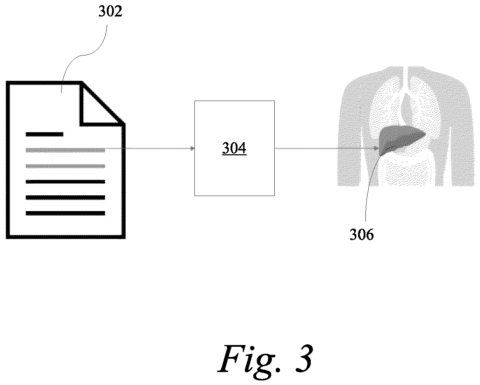

FIG. 3 is schematic diagram of the exemplary method 200 of estimating an image location from text input. The radiology report 302 of a patient is processed by the processing module 106 to extract input text. The input text is fed to the trained artificial neural network 304, which maps the input text to normalized coordinates. The corresponding location 306 in a target image of the patient is determined based on the normalized coordinates. The corresponding location 306 may be highlighted on the target image when, for example, the user selects a hyperlink inserted at the input text on the radiology report 302.

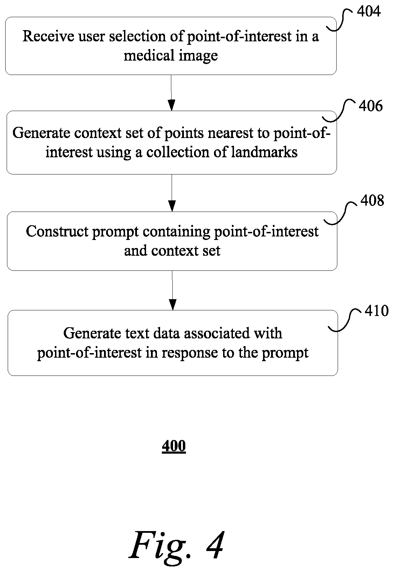

FIG. 4 is a flow chart of an exemplary method 400 of determining text data associated with a point-of-interest in a medical image. It should be understood that the steps of the method 400 may be performed in the order shown or a different order. Additional, different, or fewer steps may also be provided. Further, the method 400 may be implemented with the system 100 of FIG. 1, a different system, or a combination thereof.

At 404, processing module 106 receives a user selection of a point-of-interest in a medical image and determines normalized coordinates of the point-of-interest. The medical image may be acquired from a current patient by, for example, medical imaging device 102. The medical image may be a CT, MR, PET, SPECT or any other type of image. The user may select the point-of-interest via, for example, a user interface (e.g., graphical user interface) displayed at workstation 103.

In some implementations, processing module 106 determines the normalized coordinates of the point-of-interest. The normalized coordinates may be determined with the use of the medical image pixel (or voxel) value associated with the point-of-interest. In some implementations, a trained regression neural network may be used to estimate the normalized coordinates of the point-of-interest. The regression neural network may include, for example, an 8-layer residual network. Other types of artificial neural networks are also useful. The input of the trained regression neural network may be the voxel (or pixel) intensity value associated with the point-of-interest, and the output of the neural network is the corresponding normalized coordinates.

At 406, processing module 106 generates a context set of points nearest to the point-of-interest. The context set of points may be identified from, for example, a database 107 of landmarks. The landmarks in the database 107 may be identified in an anatomical atlas as possible points-of-interest and defined in a normalized coordinate system. As discussed previously, the normalized coordinate system is a continuous coordinate system that is scaled to a predefined bounding box. The context set of points may include a plurality of landmarks and their corresponding normalized coordinates nearest to the point-of-interest. These nearest landmarks may be determined by looking up the database of landmarks using the normalized coordinates of the point-of-interest and computing the distances between the landmarks in the database 107 and the point-of-interest to find the nearest landmarks with the shortest distances (e.g., less than a pre-determined threshold distance).

At 408, processing module 106 constructs a prompt containing the point-of-interest and the context set of points. The prompt may include the normalized coordinates and names of the context set of points and the point-of-interest. The prompt may then be sent to a trained large language model (LLM) to generate text data associated with the point-of-interest. The LLM is an artificial intelligence model that can be used for understanding and generating human-like responses after training with massive amounts of data. The LLM may include one or more artificial neural networks (e.g., transformers) that are trained using self-supervised and/or semi-supervised learning. The LLM uses deep learning techniques and has encoded anatomical knowledge to ensure better comprehension of relationships and relative locations between anatomical features. The LLM may be trained with medical health record data where anatomical information is frequently present. Such medical health record data may be obtained from an electronic health record dataset of a large number of patients (e.g., over 1 million) and include real medical images and associated radiology reports.

In some implementations, the prompt requests the LLM to use the context set of points to generate text data in association with an anatomical feature at the point-of-interest. For example, the prompt may request for a description (e.g., name, label) of the anatomical feature at the point-of-interest. An exemplary prompt is as follows: "what anatomical landmark/organ is likely to be in position [10.30, - 83.40, 9.179259], if the spatial position of the suprasternal notch is in [7.6, -71.22, 49.87], the spatial position of the common carotid artery origin (r) is in [-1.51, -43.21, 36.80], the spatial position of the aorta asc. is in [-5.77, -43.17, -17.51], the spatial position of the common carotid artery origin (l) is in [15.25, -41.54, 38.27], the spatial position of the pulmonary trunk is in [23.26, -22.09, -12.0], and the spatial position of the pulmonary artery is in [28.3936, -16.13, -19.0]?" An exemplary response is as follows: "Sternum or nearby".

At 410, a trained LLM is used to generate text data associated with the point-of-interest in response to the prompt. Processing module 106 may receive the generated text data from the trained LLM and display the text data at, for example, workstation 103. The generated text data may describe or provide semantic meaning to the point-of-interest in response to the prompt. The knowledge encoded in the LLM model is richer than the limited landmarks present in the database 107 of landmarks. This information may be useful for both report generation and information retrieval of findings for any selected point-of-interest. For example, generated text data may be compared to existing medical history of the patient to find mentions in corresponding locations.

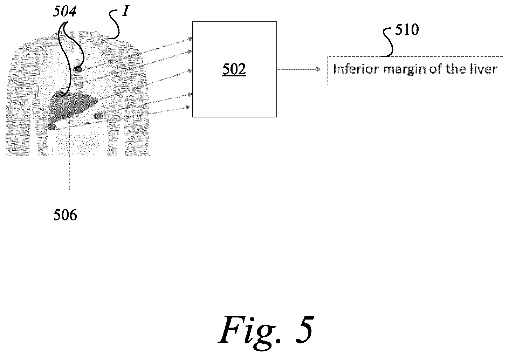

FIG. 5 is schematic diagram of the exemplary method 400 of determining text data associated with a point-of-interest in a medical image. The method employs an LLM 502 with the knowledge of human anatomy to identify anatomical landmarks using only positional information from medical image $I$. The normalization of the coordinates of the point-of-interest 504 creates the relationship between the current medical image and the atlas image with the associated landmark database. Therefore, nearby landmarks may be identified directly with distances in normalized coordinate space. Given these landmarks' positions in a normalized coordinate system, the LLM 502 may identify any randomly selected point 504 in the medical image $I$.

The position ($p$) of $n$ landmarks $l$ in the image $I$ is known in set s, wherein s = $\{l_i : p_i\}_{i=1, ..., n}$, and $i$ is an index and $n$ is a positive integer. A user may submit a request 506 that indicates a selection of a point-of-interest $r_j$ (504) on image $I$. Given the point-of-interest $r_j$ (504), $m$ closest points in the set s are selected to serve as context $c$:

$$c = \{l_i : p_i\}_{i=1, ..., m} \quad \text{------} \quad (1)$$

wherein $i$ is an index and $m$ is a positive integer.

Processing module 106 constructs a prompt containing the context set $c$ and point-of-interest $r_i$ (504). The prompt is sent to LLM 502. LLM 502 identifies the most relevant landmark $\bar{l}_j$, corresponding to the point-of-interest $r_i$ (504) given the set $c$ :

$$\bar{l}_j = LLM(c, r_i) \quad \text{------} \quad (2)$$

LLM 502 then generates text data 510 that describes the point-of-interest $r_j$. In this case, the text data "Inferior margin of the liver" is generated.

The following is a list of non-limiting illustrative embodiments disclosed herein:

Illustrative embodiment 1. A system, comprising: a non-transitory memory device for storing computer readable program code; and a processor device in communication with the non-transitory memory device, the processor device being operative with the computer readable program code to perform steps including: receiving input text and a target image of a patient,

mapping the input text into target normalized coordinates using an artificial neural network, determining a location in the target image that corresponds to the target normalized coordinates, and presenting the location in the target image.

Illustrative embodiment 2. The system of illustrative embodiment 1 wherein the steps further comprise determining the input text by splitting a radiology report into different parts using tokenization techniques to generate one or more valid tokens.

Illustrative embodiment 3. The system of any one of illustrative embodiments 1-2 wherein the steps further comprise providing a user interface that enables a user to select the input text from a radiology report.

Illustrative embodiment 4. The system of any one of illustrative embodiments 1-3 wherein the artificial neural network is trained using a training set of keywords with corresponding normalized coordinates, wherein the keywords describe anatomical features in an anatomical atlas.

Illustrative embodiment 5. The system of illustrative embodiment 4 wherein the corresponding normalized coordinates are scaled to a pre-defined bounding box.

Illustrative embodiment 6. The system of illustrative embodiment 4 wherein the corresponding normalized coordinates are determined using subtraction of pre-defined landmark coordinates.

Illustrative embodiment 7. The system of any one of illustrative embodiments 1-6 wherein determining the location in the target image that corresponds to the target normalized coordinates comprises performing a search to find the location within the target image with normalized coordinates that match the target normalized coordinates.

Illustrative embodiment 8. A method of determining text data associated with a point-of-interest, comprising: receiving a user selection of the point-of-interest in a medical image; generating a context set of points nearest to the point-of-interest; constructing a prompt containing the point-of-interest and the context set of points; and generating, by a large language model, text data associated with the point-of-interest in response to the prompt.

Illustrative embodiment 9. The method of illustrative embodiment 8 further comprising determining normalized coordinates of the point-of-interest.

Illustrative embodiment 10. The method of illustrative embodiment 9 wherein determining the normalized coordinates of the point-of-interest comprises using a trained regression neural network.

Illustrative embodiment 11. The method of any one of illustrative embodiments 8-10 wherein generating the context set of points comprises identifying the context set of points from a database of landmarks.

Illustrative embodiment 12. The method of illustrative embodiment 11 wherein the database of landmarks are defined in a normalized coordinate system.

Illustrative embodiment 13. The method of illustrative embodiment 12 wherein identifying the context set of points comprises looking up the database of landmarks using normalized coordinates of the point-of-interest and computing distances between one or more landmarks in the database of landmarks and the point-of-interest to find the context set of points nearest to the point-of-interest.

Illustrative embodiment 14. The method of any one of illustrative embodiments 8-13 wherein constructing the prompt comprises constructing the prompt that requests for a description of an anatomical feature at the point-of-interest.

Illustrative embodiment 15. The method of illustrative embodiment 14 wherein the prompt requests for the description of the anatomical feature at the point-of-interest given normalized coordinates of the point-of-interest and the context set of points.

Illustrative embodiment 16. The method of any one of illustrative embodiments 8-15 wherein the large language model is trained with medical health record data.

Illustrative embodiment 17. One or more non-transitory computer-readable media comprising computer-readable instructions, that when executed by a processor device, cause the processor device to perform steps comprising: receiving a user selection of a point-of-interest in a medical image; determining normalized coordinates of the point-of-interest; generating a context set of points nearest to the point-of-interest, wherein the context set of points are identified from a database of landmarks defined in a normalized coordinate system; constructing a prompt containing the point-of-interest and the context set of points; and generating, by a large language model, text data associated with the point-of-interest in response to the prompt.

Illustrative embodiment 18. The one or more non-transitory computer-readable media of illustrative embodiment 17 wherein constructing the prompt comprises constructing the prompt that requests for a description of an anatomical feature at the point-of-interest.

Illustrative embodiment 19. The one or more non-transitory computer-readable media of any one of illustrative embodiments 17-18 wherein the prompt contains the normalized coordinates of the point-of-interest and the context set of points.

Illustrative embodiment 20. The one or more non-transitory computer-readable media of any one of illustrative embodiments 17-19 wherein determining the normalized coordinates of the point-of-interest comprises using a trained regression neural network.

While the present framework has been described in detail with reference to exemplary embodiments, those skilled in the art will appreciate that various modifications and substitutions can be made thereto without departing from the spirit and scope of the invention as set forth in the appended claims. For example, elements and/or features of different exemplary embodiments may be combined with each other and/or substituted for each other within the scope of this disclosure and appended claims.

**Claims**

1.  A system, comprising:

    a non-transitory memory device for storing computer readable program code; and
    a processor device in communication with the non-transitory memory device, the processor device being operative with the computer readable program code to perform steps including

    receiving input text and a target image of a patient,
    mapping the input text into target normalized coordinates using an artificial neural network,
    determining a location in the target image that corresponds to the target normalized coordinates, and
    presenting the location in the target image.

2.  The system of claim 1 wherein the steps further comprise determining the input text by splitting a radiology report into different parts using tokenization techniques to generate one or more valid tokens.

3.  The system of claim 1 or 2 wherein the steps further comprise providing a user interface that enables a user to select the input text from a radiology report.

4.  The system of one of claims 1 to 3 wherein the artificial neural network is trained using a training set of keywords with corresponding normalized coordinates, wherein the keywords describe anatomical features in an anatomical atlas.

5. The system of claim 4 wherein the corresponding normalized coordinates are scaled to a pre-defined bounding box.

6. The system of claim 4 wherein the corresponding normalized coordinates are determined using subtraction of pre-defined landmark coordinates.

7. The system of one of claims 1 to 6 wherein determining the location in the target image that corresponds to the target normalized coordinates comprises performing a search to find the location within the target image with normalized coordinates that match the target normalized coordinates.

8. A method of determining text data associated with a point-of-interest, comprising:

   receiving a user selection of the point-of-interest in a medical image;
   generating a context set of points nearest to the point-of-interest;
   constructing a prompt containing the point-of-interest and the context set of points; and
   generating, by a large language model, text data associated with the point-of-interest in response to the prompt.

9. The method of claim 8 further comprising determining normalized coordinates of the point-of-interest.

10. The method of claim 9 wherein determining the normalized coordinates of the point-of-interest comprises using a trained regression neural network.

11. The method of one of claims 8 to 10 wherein generating the context set of points comprises identifying the context set of points from a database of landmarks.

12. The method of claim 11 wherein the database of landmarks are defined in a normalized coordinate system.

13. The method of claim 12 wherein identifying the context set of points comprises looking up the database of landmarks using normalized coordinates of the point-of-interest and computing distances between one or more landmarks in the database of landmarks and the point-of-interest to find the context set of points nearest to the point-of-interest.

14. The method of one of claims 8 to 13 wherein constructing the prompt comprises constructing the prompt that requests for a description of an anatomical feature at the point-of-interest.

15. The method of claim 14 wherein the prompt requests for the description of the anatomical feature at the point-of-interest given normalized coordinates of the point-of-interest and the context set of points.

16. The method of one of claims 8 to 15 wherein the large language model is trained with medical health record data.

17. One or more non-transitory computer-readable media comprising computer-readable instructions, that when executed by a processor device, cause the processor device to perform steps comprising:

   receiving a user selection of a point-of-interest in a medical image;
   determining normalized coordinates of the point-of-interest;
   generating a context set of points nearest to the point-of-interest, wherein the context set of points are identified from a database of landmarks defined in a normalized coordinate system;
   constructing a prompt containing the point-of-interest and the context set of points; and
   generating, by a large language model, text data associated with the point-of-interest in response to the prompt.

18. The one or more non-transitory computer-readable media of claim 17 wherein constructing the prompt comprises constructing the prompt that requests for a description of an anatomical feature at the point-of-interest.

19. The one or more non-transitory computer-readable media of claim 17 or 18 wherein the prompt contains the normalized coordinates of the point-of-interest and the context set of points.

20. The one or more non-transitory computer-readable media of one of claims 17 to 19 wherein determining the normalized coordinates of the point-of-interest comprises using a trained regression neural network.

101

121

Processor Device
104

Non-transitory
computer-readable
Media
105

Processing
Module
106

107

Display Device

108

Input Devices

110

132

Medical
imaging device
102

144

Workstation

103

100

*Fig. 1*

Receive input text and target image ⌒ 204

Map input text into target normalized coordinates using artificial neural network ⌒ 206

Determine location in target image corresponding to the target normalized coordinates ⌒ 208

Present the location in the target image ⌒ 210

**200**

*Fig. 2*

*Fig. 3*

Receive user selection of point-of-interest in a medical image — 404

Generate context set of points nearest to point-of-interest using a collection of landmarks — 406

Construct prompt containing point-of-interest and context set — 408

Generate text data associated with point-of-interest in response to the prompt — 410

**400**

# *Fig. 4*

*504*  I  510

502

Inferior margin of the liver

506

*Fig. 5*

**EP 4 542 562 A2**

## REFERENCES CITED IN THE DESCRIPTION

**Patent documents cited in the description**

- US 63585743